# EUROPEAN PATENT APPLICATION

(11) **EP 4 738 196 A1**
(43) Date of publication of application: **06.05.2026**
(21) Application number: 25210934.3
(22) Date of filing: 24.10.2025
(51) Int. Cl.: G06N 3/045, G06N 3/0455, G06N 3/0464, G06N 3/047, G06N 3/088

(54) **CONTROL PROGRAM, CONTROL METHOD, AND INFORMATION PROCESSING DEVICE**

(30) Priority: 30.10.2024 JP 2024191213
(71) Applicant: FUJITSU LIMITED, Kawasaki-shi, Kanagawa 211-8588 (JP)
(72) Inventor: YOSHIKAWA, Hiyori, Kawasaki-shi, Kanagawa, 211-8588 (JP); TOMA, Mitsunori, Kawasaki-shi, Kanagawa, 211-8588 (JP); YAMAZAKI, Kimihiro, Kawasaki-shi, Kanagawa, 211-8588 (JP); WADA, Yuichiro, Kawasaki-shi, Kanagawa, 211-8588 (JP); WADA, Mutsuyo, Kawasaki-shi, Kanagawa, 211-8588 (JP); WAIDA, Hiroki, Kawasaki-shi, Kanagawa, 211-8588 (JP); ISHII, Yoshiyuki, Kawasaki-shi, Kanagawa, 211-8588 (JP); KATOH, Takashi, Kawasaki-shi, Kanagawa, 211-8588 (JP); NAKAGAWA, Akira, Kawasaki-shi, Kanagawa, 211-8588 (JP)
(74) Representative: Hoffmann Eitle

(57) **Abstract**

A control program that causes a computer to execute a process, the process including: generating an intermediate representation corresponding to input data, the intermediate representation being generated using a trained model; training a distribution of latent representations corresponding to the generated intermediate representation, using a predetermined encoder generating the latent representation from the intermediate representation and a predetermined decoder corresponding to the predetermined encoder and generating an intermediate representation different from the intermediate representation; selecting, from the trained distribution, a sample of the latent representation based on a probability distribution; generating, using the predetermined decoder, a new intermediate representation corresponding to the selected sample; and generating, using the trained model, output data corresponding to the generated new intermediate representation.

## Description

### FIELD

The embodiments discussed herein are related to a control program, a control method, and an information processing device.

### BACKGROUND

Conventionally, deep learning models that handle sequence information, such as Transformer models, have demonstrated high performance in understanding input information and predicting structured outputs (for example, refer to, Vaswani, Ashish, et al, "Attention is all you need." Advances in neural information processing systems 30 (2017)). Large-scale trained models with high expressive capabilities are also known, such as AlphaFold2, which predicts protein structures (or example, refer to, Jumper, John, et al, "Highly accurate protein structure prediction with AlphaFold." Nature 596.7873 (2021): 583-589). There are cases where it is desirable to take advantage of the expressive capabilities of such trained models to generate high-quality, diverse outputs without making changes to the parameters of the trained model.

### SUMMARY

It is an object in one aspect of the embodiments to at least solve the above problems in the conventional technologies.

According to an aspect of an embodiment, a control program that causes a computer to execute a process, the process including: generating an intermediate representation corresponding to input data, the intermediate representation being generated using a trained model; training a distribution of latent representations corresponding to the generated intermediate representation, using a predetermined encoder generating the latent representation from the intermediate representation and a predetermined decoder corresponding to the predetermined encoder and generating an intermediate representation different from the intermediate representation; selecting, from the trained distribution, a sample of the latent representation based on a probability distribution; generating, using the predetermined decoder, a new intermediate representation corresponding to the selected sample; and generating, using the trained model, output data corresponding to the generated new intermediate representation.

### BRIEF DESCRIPTION OF DRAWINGS

Fig. 1 is an explanatory diagram depicting an example of a control method according to an embodiment.
Fig. 2 is an explanatory diagram depicting an example of a system configuration of an information processing system 200.
Fig. 3 is a block diagram depicting an example of a hardware configuration of a controller 201.
Fig. 4 is an explanatory diagram depicting a specific example of input data 400.
Fig. 5 is an explanatory diagram depicting a specific example of an intermediate representation 500.
Fig. 6 is a block diagram depicting an example of a functional configuration of the controller 201.
Fig. 7 is an explanatory diagram depicting a first operation example of the controller 201.
Fig. 8 is an explanatory diagram depicting the first operation example of the controller 201.
Fig. 9 is an explanatory diagram depicting a second operation example of the controller 201.
Fig. 10 is an explanatory diagram depicting the second operation example of the controller 201.
Fig. 11 is an explanatory diagram depicting the second operation example of the controller 201.
Fig. 12 is an explanatory diagram depicting the second operation example of the controller 201.
Fig. 13 is an explanatory diagram depicting a third operation example of the controller 201.
Fig. 14 is an explanatory diagram depicting the third operation example of the controller 201.
Fig. 15 is an explanatory diagram depicting a fourth operational example of the controller 201.
Fig. 16 is a flowchart depicting an example of a procedure of a training process.
Fig. 17 is a flowchart depicting an example of a procedure of a generation process.

### DESCRIPTION OF EMBODIMENTS

First, problems associated with the conventional techniques are discussed. In the conventional techniques, when some kind of operation is attempted to be perform on an intermediate representation of a trained model to change the output, the range of operation of the intermediate representation that corresponds to valid output is not clear, making it difficult to determine what kind of operation will result in valid output.

Embodiments of a control program, a control method, and an information processing device according to the present disclosure are described in detail with reference to the accompanying drawings.

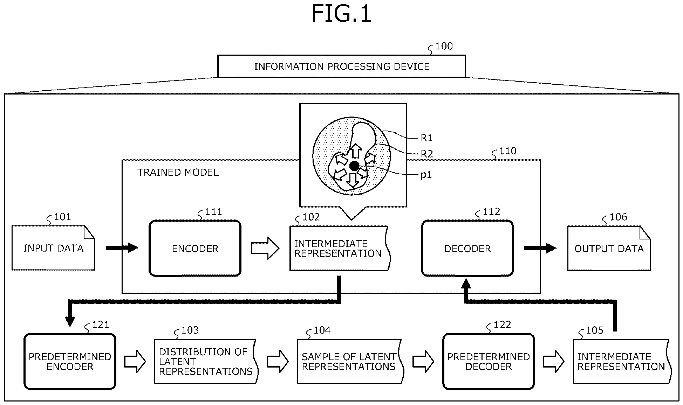

Fig. 1 is an explanatory diagram depicting an example of a control method according to an embodiment. The information processing device 100 is a computer for controlling operations on an intermediate representation corresponding to input data to a trained model. The information processing device 100 is, for example, a server or a personal computer (PC).

Here, the trained model is a machine learning model trained by machine learning such as deep learning. Deep learning is also called deeplayer learning. The trained model is, for example, information that combines trained parameters and an algorithm for deriving output data corresponding to input data based on the trained parameters.

When the trained model receives input data according to the algorithm, the trained model derives output data by applying the trained parameters to the input data. For example, the trained model includes an encoder that converts the input data into an intermediate representation and a decoder that converts the intermediate representation into output data. For example, the trained model generates output data by converting the input data into an intermediate representation using the encoder and then converting the converted intermediate representation into output data using the decoder.

The intermediate representation is information obtained by extracting features from input data. For example, an intermediate representation that is a vector sequence is obtained by extracting features from input data that is sequence information. The intermediate representation is manipulated to obtain new output data for the input data. For example, it is possible to manipulate the intermediate representation by adding a small value to the intermediate representation that is a vector sequence to change the value of the vector sequence.

Examples of trained models include the Transformer model and AlphaFold2. The Transformer model has a function of taking sequence information representing a sentence as input data and outputting sequence information representing another sentence as output data. AlphaFold2 has a function of taking amino acid sequence information as input data and outputting output data representing the structure (three-dimensional structure) of a protein. The Transformer model and AlphaFold2 are large-scale deep learning models with high expressive capabilities.

For details about the Transformer model, refer to, for example, Vaswani, Ashish, et al, "Attention is all you need." mentioned above or Radford, Alec, et al. "Language models are unsupervised multitask learners." OpenAI blog 1.8 (2019): 9. For details about AlphaFold2, refer to, for example, Jumper, John, et al, "Highly accurate protein structure prediction with AlphaFold." mentioned above.

There is a demand for leveraging the expressive capabilities of such trained models to generate high-quality, diverse outputs without making changes to parameters of the trained model. For example, there may be an instance which use of AlphaFold2 to enumerate the polymorphisms that an input sequence may take is desired. Also, there may be an instance in which use of a Transformer model for text generation to generate diverse sentences is desired.

Here, the intermediate representation of a large-scale model abstractly captures important features of the data, and is expected to be suitable for making meaningful changes to the output while maintaining the essence of the input data. For this reason, it is conceivable to generate new output data for input data by manipulating the intermediate representation of a trained model.

However, there is a problem in that it is difficult to manipulate the intermediate representation in a way that produces valid output. For example, a Transformer model cannot explicitly calculate the probability distribution of an intermediate representation. Therefore, when some kind of operation is performed on the intermediate representation in an attempt to change an output, it is unclear what range of operations on the intermediate representation is necessary to obtain an output that is deemed valid based on the data distribution of the training data used to train the trained model. Therefore, it is unclear what range of operations are to be performed on the intermediate representation to obtain an output that is deemed valid, making it difficult to appropriately manipulate the intermediate representation. Here, the operation range of the intermediate representation will be explained with reference to Fig. 1.

In Fig. 1, a black circle p1 indicates an intermediate representation converted from input data. Regions R1 and R2 both represent possible ranges of intermediate representations. Region R1 represents, for example, a region that has generalized to a certain extent through training. Region R2 represents, for example, a region that includes only intermediate representations that correspond to valid outputs. Region R2 corresponds, for example, to the distribution of intermediate representations corresponding to input data, which is various training data used to train the trained model.

Because region R1 includes intermediate representations corresponding to invalid output, while there is a possibility of obtaining valid output, there is also a possibility of obtaining invalid output. Therefore, in order to obtain valid output, it is preferable to control operations on the intermediate representation so that the intermediate representation falls within region R2. However, because region R2 cannot be explicitly obtained, it is unclear how to, for example, control operations on the intermediate representation to obtain valid output.

For example, as a result of manipulating the intermediate representation, the intermediate representation may extend beyond region R2, resulting in an intermediate representation that corresponds to a location outside the data distribution of the training data used to train the trained model. In this case, the output estimated based on the intermediate representation may be unreliable and outside the data distribution of the training data.

Therefore, the present embodiment describes a control method that may control operations on the intermediate representation in a direction that results in valid output.

In Fig. 1, an information processing device 100 has a trained model 110. The trained model 110 includes an encoder 111 that converts input data into an intermediate representation and a decoder 112 that converts the intermediate representation into output data. In the example depicted in Fig. 1, data input to the trained model 110 is "input data 101." For example, when the trained model 110 is "AlphaFold2," the encoder 111 corresponds to a Transformer encoder. The decoder 112 corresponds to a Transformer decoder.

The information processing device 100 also includes a predetermined encoder 121. The predetermined encoder 121 has, for example, a function of converting an intermediate representation into a latent representation. The predetermined encoder 121 is, for example, an encoder used in a variational autoencoder (VAE) technique. The information processing device 100 also includes a predetermined decoder 122 that corresponds to the predetermined encoder 121. The predetermined decoder 122 has a function of converting an input latent representation into an intermediate representation. The predetermined decoder 122 is, for example, a decoder used in the VAE technique.

As indicated below, the information processing device 100 generates new output data 106 corresponding to the input data 101 by manipulating an intermediate representation 102 corresponding to the input data 101. For example, the operation includes generating another intermediate representation 105 based on the intermediate representation 102 corresponding to the input data 101. The output data 106 is information different from the output data obtained by directly converting the intermediate representation 102 using the decoder 112.

(1-1) The information processing device 100 generates the intermediate representation 102 corresponding to the input data 101 using the trained model 110. The input data 101 is, for example, training data used when training the trained model 110. For example, the information processing device 100 generates the intermediate representation 102 corresponding to the input data 101 by converting the input data 101 using the encoder 111.

(1-2) The information processing device 100 trains a distribution 103 of latent representations corresponding to the generated intermediate representation 102 according to the predetermined encoder 121 and the predetermined decoder 122. The information processing device 100, for example, trains the distribution 103, which is a probability distribution of latent representations projected from the intermediate representation 102, according to the predetermined encoder 121 and the predetermined decoder 122. For example, the distribution 103 represents the probability that each of multiple latent representations is possible. This allows the information processing device 100 to obtain the distribution 103 of latent representations corresponding to the intermediate representation 102, which corresponds to a valid range for manipulating the intermediate representation 102.

(1-3) The information processing device 100 selects a sample 104 of latent representations from the trained distribution 103. For example, the information processing device 100 selects the sample 104 of latent representations from the trained distribution 103 based on a probability distribution. The sample 104 is obtained, for example, by sampling data according to the probability distribution. This allows the information processing device 100 to obtain the sample 104 of latent representations that will serve as the basis for a valid, new intermediate representation 105.

(1-4) The information processing device 100 generates the new intermediate representation 105 corresponding to the selected sample 104 using the predetermined decoder 122. For example, the information processing device 100 generates the new intermediate representation 105 corresponding to the selected sample 104 by converting the selected sample 104 using the predetermined decoder 122. This allows the information processing device 100 to manipulate the intermediate representation 102 within an operation range based on the distribution 103 of the latent representation, thereby obtaining the valid, new intermediate representation 105.

(1-5) The information processing device 100 generates the output data 106 corresponding to the generated new intermediate representation 105 using the trained model 110. For example, the information processing device 100 converts the generated new intermediate representation 105 using the decoder 112 to generate the output data 106 corresponding to the generated new intermediate representation 105. This allows the information processing device 100 to obtain valid output data 106.

As described, the information processing device 100 may control operations on the intermediate representation 102 in a direction that results in the valid output data 106. The information processing device 100 may, for example, apply operations to the intermediate representation 102 within an operation range based on the distribution 103 of latent representations, thereby obtaining the valid, new intermediate representation 105 and the valid output data 106. Therefore, the information processing device 100 may obtain the valid output data 106 more efficiently than when applying random operations to the intermediate representation 102.

Here, while a case where the functions of the information processing device 100 are implemented by a single computer has been described, this is not a limitation. For example, the functions of the information processing device 100 may be implemented by cooperation between multiple computers. For example, the functions of the information processing device 100 may be implemented on a cloud.

Next, an example of a system configuration of an information processing system 200 including the information processing device 100 depicted in Fig. 1 will be described. Here, an example will be described in which the information processing device 100 depicted in Fig. 1 is applied to a controller 201 in the information processing system 200.

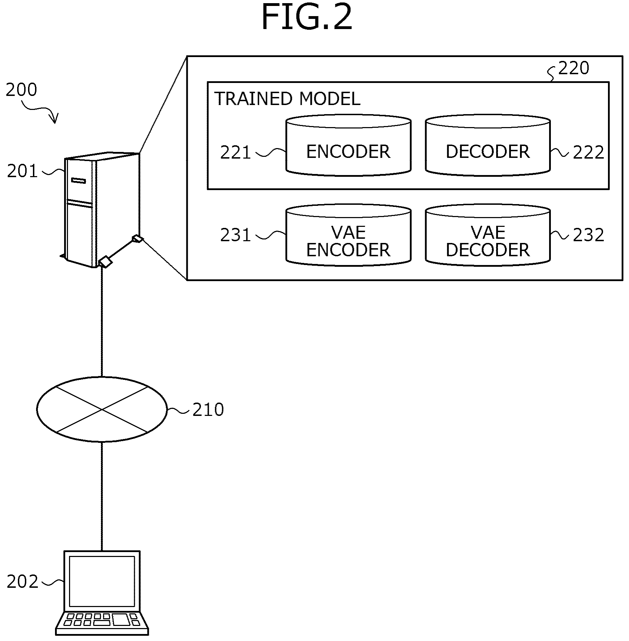

Fig. 2 is an explanatory diagram depicting an example of the system configuration of the information processing system 200. In Fig. 2, the information processing system 200 includes the controller 201 and a client device 202. In the information processing system 200, the controller 201 and the client device 202 are coupled via a wired or wireless network 210. The network 210 is, for example, the Internet, a local area network (LAN), or a wide area network (WAN).

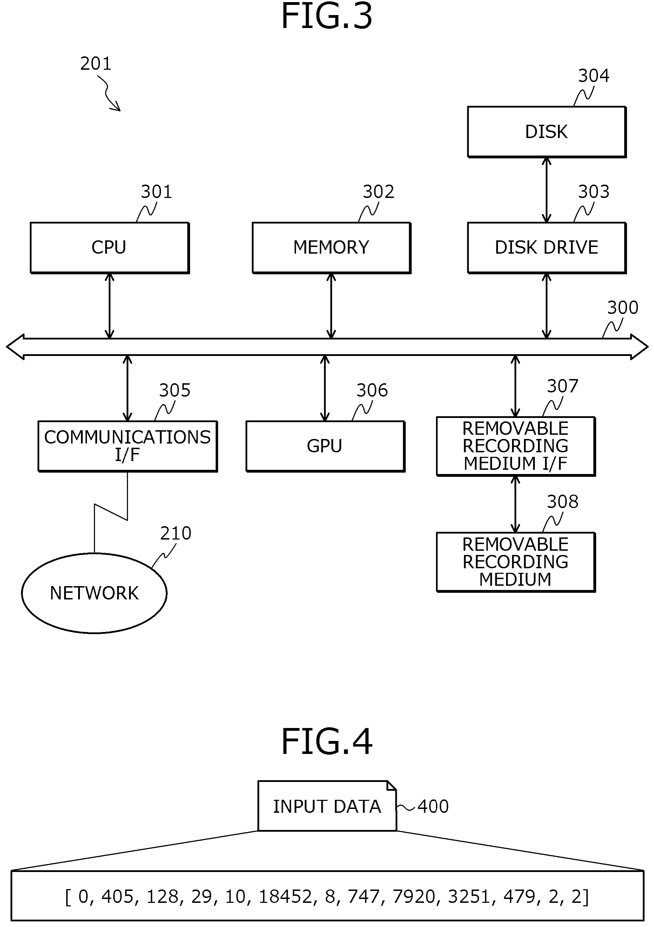

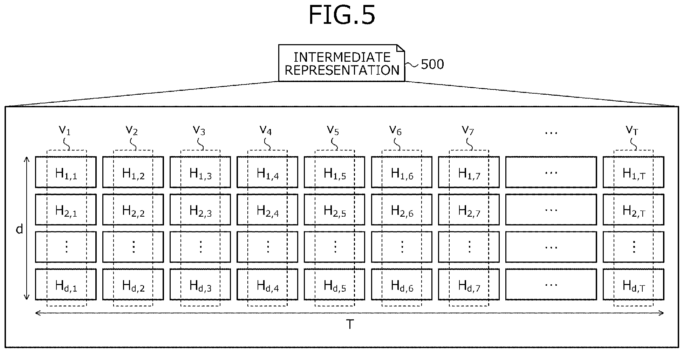

Here, the controller 201 is a computer that controls operations on an intermediate representation corresponding to input data to a trained model 220. Specific examples of input data will be described later with reference to Fig. 4. Specific examples of intermediate representations will be described later with reference to Fig. 5.

The controller 201 has the trained model 220. The trained model 220 is, for example, a trained deep learning model such as a Transformer model or AlphaFold2. The trained model 220 includes an encoder 221 and a decoder 222. The controller 201 may train a deep learning model that becomes the trained model 220, for example, using training data. The controller 201 also includes a VAE encoder 231 and a VAE decoder 232.

The trained model 110 depicted in Fig. 1 corresponds, for example, to the trained model 220. The encoder 111 depicted in Fig. 1 corresponds, for example, to the encoder 221. The decoder 112 depicted in Fig. 1 corresponds, for example, to the decoder 222. The predetermined encoder 121 depicted in Fig. 1 corresponds, for example, to the VAE encoder 231. The predetermined decoder 122 depicted in Fig. 1 corresponds, for example, to the VAE decoder 232.

The controller 201 receives, from the client device 202, a processing request requesting the generation of various output data based on input data. The processing request includes, for example, input data. The input data is, for example, training data used when training the trained model 220. In response to the processing request, the controller 201 generates multiple pieces of output data based on the input data using the trained model 220, the encoder 221, and the decoder 222. The controller 201 generates multiple pieces of output data by using, for example, the trained model 220 to train the distribution of latent representations using the input data according to the VAE encoder 231 and the VAE decoder 232. The controller 201 transmits the generated multiple pieces of output data to the client device 202. The controller 201 is, for example, a server or a PC.

The client device 202 is a computer used by a user of the information processing system 200. The user may, for example, wish to predict the structure of a protein from an amino acid sequence or generate another sentence from a given sentence. The other sentence, for example, may be a translated sentence. The client device 202 generates a processing request based on user input via an input device (not depicted) and transmits the processing request to the controller 201. The client device 202 receives multiple pieces of output data from the controller 201 and outputs the received output data so that the user may refer to the data. The client device 202 may be, for example, a PC, a tablet terminal, or a smartphone.

Here, while a case where the controller 201 and the client device 202 are different devices has been described, this is not a limitation. For example, the controller 201 may have the functionality of the client device 202 and operate as the client device 202. The information processing system 200 may include multiple client apparatuses 202.

The information processing system 200, for example, may be applied to a case where it is desired to present multiple pieces of output data representing a protein structure to a user based on input data representing an amino acid sequence. Furthermore, for example, the information processing system 200 may be applied to cases where it is desired to present to a user, based on input data representing a certain sentence, multiple output data representing translations of the sentence.

Next, an example of a hardware configuration of the controller 201 is described.

Fig. 3 is a block diagram depicting an example of the hardware configuration of the controller 201. In Fig. 3, the controller 201 has a central processing unit (CPU) 301, a memory 302, a disk drive 303, and a disk 304. The controller 201 further has a communications interface (I/F) 305, a graphics processing unit (GPU) 306, a removable-recording medium I/F 307, and a removable-recording medium 308. The components are coupled to each other by a bus 300.

Here, the CPU 301 governs overall control of the controller 201. The GPU 306 performs computational processing such as image processing and natural language processing. The CPU 301 and/or the GPU 306 may have multiple cores. The memory 302, for example, includes read-only memory (ROM), random access memory (RAM), and the like. Programs stored in the memory 302are loaded onto the CPU 301, whereby encoded processes are executed by the CPU 301.

The disk drive 303, under the control of the CPU 301, controls the reading and writing of data with respect to the disk 304. The disk 304 stores data written thereto under the control of the disk drive 303. The disk 304 is, for example, a magnetic disk, an optical disk, or the like.

The communications I/F 305 is coupled to a network 210 through a communications line and is coupled to external computers via the network 210. An external computer, for example, is the client device 202 depicted in Fig. 2. Further, the communications I/F 305 administers an internal interface with the network 210 and controls the input and output of data from external computers. The communications I/F 305, for example, is a modem, a LAN adapter, or the like.

The removable-recording medium I/F 307, under the control of the CPU 301, controls the reading and writing of data with respect to the removable-recording medium 308. The removable-recording medium 308 stores data written thereto under the control of the removable-recording medium I/F 307. The removable-recording medium 308, for example, is a compact disc (CD)-ROM, a digital versatile disk (DVD), a universal serial bus (USB) memory, or the like.

In addition to the components above, the controller 201 may have, for example, an input device, a display, a printer, a scanner, a microphone, a speaker, or the like. Further, among the components described above, the controller 201 may omit, for example, the GPU 306, the removable-recording medium I/F 307, and the removable-recording medium 308.

The hardware configuration example of the client device 202 is, for example, similar to the hardware configuration example of the controller 201 depicted in Fig. 3 and therefore, a description thereof is omitted. The client device 202 may also include, for example, an input device, a display, or the like, in addition to the components depicted in Fig. 3.

Next, a specific example of input data 400 input to the trained model 220 depicted in Fig. 2 will be described with reference to Fig. 4. Here, a case where the trained model 220 is a "Transformer model" is described as an example, and a case where sequence information representing a sentence is input to the trained model 220 as the input data 400 is assumed.

Fig. 4 is an explanatory diagram depicting a specific example of the input data 400. In Fig. 4, the input data 400 is sequence information indicating a token ID string representing a sentence. A token corresponds to a sentence (text) divided into units such as words, subwords, or symbols. A token ID is an identifier that identifies a token.

The input data 400 corresponds to the input text "it's a charming and often affecting journey.", to which preprocessing such as tokenization has been applied. Preprocessing includes, for example, replacing units such as words, subwords, or symbols with token IDs. Preprocessing may be performed, for example, by the controller 201, or by a computer other than the controller 201. The other computer may be, for example, the client device 202. In the following description, the number of token IDs is referred to as the "length T of the sequence information."

Next, with reference to Fig. 5, a specific example of an intermediate representation 500 obtained by converting the input data 400 input to the trained model 220 depicted in Fig. 2 using the encoder 221 will be described.

Fig. 5 is an explanatory diagram depicting a specific example of the intermediate representation 500. In Fig. 5, the intermediate representation 500 is information converted from the input data 400 depicted in Fig. 4 by extracting features from the input data 400 using the encoder 221. The intermediate representation 500 is a vector sequence in which vectors v₁ to v_{T} corresponding to each token ID are arranged for the length T of the sequence information. Each of the vectors v₁ to v_{T} is a d-dimensional vector. Furthermore, H_{i,j} is the j-th component of the i-th vector vᵢ.

Although not depicted in the figure, for example, when the trained model 220 is "AlphaFold2," the input data is amino acid sequence information. The intermediate representation includes a single representation and a pair representation. The single representation is a vector sequence. The pair representation is T×T×d-dimensional array information that represents the similarity between vectors in the sequence.

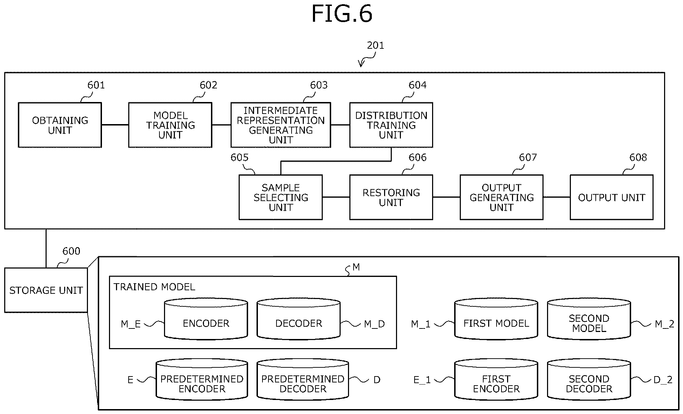

Next, an example of a functional configuration of the controller 201 will be described with reference to Fig. 6.

Fig. 6 is a block diagram depicting an example of the functional configuration of the controller 201. The controller 201 includes a storage unit 600, an obtaining unit 601, a model training unit 602, an intermediate representation generating unit 603, a distribution training unit 604, a sample selecting unit 605, a restoring unit 606, an output generating unit 607, and an output unit 608.

The storage unit 600 is implemented by a storage device such as the memory 302 or the disk 304 depicted in Fig. 3. Below, while a case where the storage unit 600 is included in the controller 201 will be described, this is not a limitation. For example, the storage unit 600 may be included in an external device different from the controller 201. In this case, for example, the contents stored in the storage unit 600 may be accessible from the controller 201 via the network 210.

The obtaining unit 601 to the output unit 608 function as an example of a control unit. For example, functions of the obtaining unit 601 to the output unit 608 are implemented by causing the CPU 301 to execute programs stored in storage devices such as the memory 302, the disk 304, and the removable recording medium 308, or by using the communications I/F 305 and the GPU 306. The processing results of each functional unit are stored to storage devices such as the memory 302 and the disk 304 depicted in Fig. 3.

The storage unit 600 stores various types of information referenced or updated during the processing by the functional units. The storage unit 600 stores, for example, a trained model. The trained model M includes, for example, an encoder M_E that converts input data into an intermediate representation and a decoder M_D that converts the intermediate representation into output data. For example, the trained model M may be a deep learning model that takes an amino acid sequence as input data and outputs output data representing a protein structure. For example, the trained model M may be a model that takes sequence information representing a sentence as input data and outputs sequence information representing another sentence as output data. The trained model M is obtained by, for example, the obtaining unit 601. The trained model M is generated by, for example, the model training unit 602.

The storage unit 600 stores, for example, a predetermined encoder E that generates a latent representation and a predetermined decoder D that corresponds to the predetermined encoder E. The predetermined encoder E converts, for example, an intermediate representation into a latent representation. The predetermined encoder E is an encoder in the VAE technique. The predetermined decoder D converts a latent representation into an intermediate representation. The predetermined decoder D is a decoder in the VAE technique. The template of the predetermined encoder E and the template of the predetermined decoder D are obtained by, for example, the obtaining unit 601. The predetermined encoder E and the predetermined decoder D are trained by, for example, the distribution training unit 604.

Specific patterns of combinations of the predetermined encoder E and the predetermined decoder D may be, for example, a first pattern, a second pattern, or a third pattern indicated below. In the first pattern, the predetermined encoder E includes a first generator that generates a first vector by fully coupling multiple vectors representing the intermediate representation and then multiplying the vectors by a first weight matrix, and a first multilayer perceptron that generates a latent representation corresponding to the generated first vector. In the first pattern, the predetermined decoder D includes a second multilayer perceptron that generates a second vector corresponding to the latent representation, and a second generator that restores multiple vectors representing the intermediate representation from a third vector obtained by multiplying the generated second vector by a second weight matrix.

In the second pattern, the predetermined encoder E is a model that generates a latent representation by repeatedly performing a convolution operation on the intermediate representation and identifies parameters that represent the distribution of the latent representation. In the second pattern, the predetermined decoder D is a model that generates an intermediate representation by repeatedly performing a deconvolution operation on the latent representation.

In the third pattern, the predetermined encoder E includes repeating a convolution operation on the intermediate representation multiple times. The predetermined encoder E is a model that identifies a first parameter that represents the distribution of each of multiple hierarchical latent representations according to the results of each convolution operation. In the third pattern, the predetermined decoder D performs a deconvolution operation on each of the multiple latent representations. The predetermined decoder D is a model that fixes a second parameter representing the distribution of the lowest-level latent representations and identifies second parameters representing the distribution of each latent representation other than the lowest-level latent representations.

The obtaining unit 601 obtains various types of information used in the processing by the functional units. The obtaining unit 601 stores the obtained various types of information to the storage unit 600 or outputs the information to the functional units. The obtaining unit 601 may also output various types of information stored in the storage unit 600 to the functional units. The obtaining unit 601 obtains various types of information based on, for example, a user's operation input. The obtaining unit 601 may receive various types of information from, for example, a device other than the controller 201.

The obtaining unit 601 obtains, for example, a processing request requesting the generation of output data. The processing request includes, for example, input data. The input data is, for example, training data used during training of the trained model. The processing request may include the trained model M. The processing request may include a template for a predetermined encoder E and a template for a predetermined decoder D. For example, the obtaining unit 601 obtains the processing request by receiving the processing request from another computer. The other computer is, for example, the client device 202. For example, the obtaining unit 601 may obtain the processing request by receiving input of the processing request based on user operation input via an input device (not depicted).

The obtaining unit 601 obtains, for example, input data. For example, the obtaining unit 601 obtains the input data by extracting the input data from the processing request. For example, the obtaining unit 601 may obtain the input data by receiving the input data from another computer. The other computer is, for example, the client device 202. For example, the obtaining unit 601 may obtain the input data by receiving input of the input data based on user operation input via an input device (not depicted).

The obtaining unit 601 obtains, for example, the trained model M. For example, the obtaining unit 601 obtains the trained model M by extracting the trained model M from the processing request. For example, the obtaining unit 601 may obtain the trained model M by receiving the trained model M from another computer. The other computer is, for example, the client device 202. For example, the obtaining unit 601 may obtain the trained model M by receiving input of the trained model M based on user operation input via an input device (not depicted).

The obtaining unit 601 obtains, for example, a template for a predetermined encoder E and a template for a predetermined decoder D. For example, the obtaining unit 601 obtains the template for the predetermined encoder E and the template for the predetermined decoder D by extracting the templates from the processing request. For example, the obtaining unit 601 may obtain the template for the predetermined encoder E and the template for the predetermined decoder D by receiving the templates from another computer. The other computer is, for example, the client device 202. For example, the obtaining unit 601 may obtain the template for the predetermined encoder E and the template for the predetermined decoder D by receiving input of the template for the predetermined encoder E and the template for the predetermined decoder D based on a user's operational input via an input device (not depicted).

The obtaining unit 601 may receive a start trigger for starting the processing by any of the functional units. The start trigger may be, for example, a predetermined operational input by the user. The start trigger may be, for example, reception of predetermined information from another computer. The start trigger may be, for example, output of predetermined information by any of the functional units. For example, the obtaining unit 601 may regard the obtaining of a processing request as a start trigger for starting the processing by the model training unit 602, the intermediate representation generating unit 603, the distribution training unit 604, the sample selecting unit 605, the restoring unit 606, and the output generating unit 607.

The model training unit 602 generates a trained model M. The model training unit 602 generates a trained model M based on, for example, training data. This enables the model training unit 602 to generate output data even when an external trained model M is not prepared.

The intermediate representation generating unit 603 generates an intermediate representation corresponding to the input data using the trained model M. For example, the intermediate representation generating unit 603 generates an intermediate representation corresponding to the input data by converting the input data into an intermediate representation using an encoder M_E. This allows the intermediate representation generating unit 603 to obtain an intermediate representation that extracts the features of the input data.

The distribution training unit 604 trains the distribution of latent representations corresponding to the generated intermediate representation according to a predetermined encoder E and a predetermined decoder D. For example, the distribution training unit 604 employs the training data used when training the trained model M as input data. For example, the distribution training unit 604 sets an objective function. The objective function includes, for example, the likelihood of output data corresponding to the input data, the reconstruction error of the intermediate representation corresponding to the input data, and the KL divergence related to the distribution of latent representations. The distribution training unit 604 uses, for example, the objective function to train the distribution of latent representations according to a predetermined encoder E and a predetermined decoder D.

For example, it is possible that the predetermined encoder E and the predetermined decoder D are of a first pattern. In this case, the distribution training unit 604, for example, uses the objective function to train the predetermined encoder E and the predetermined decoder D and thereby train the distribution of latent representations corresponding to the generated intermediate representation. As a result, the distribution training unit 604 may obtain a distribution of latent representations corresponding to the intermediate representation, distribution of latent representations corresponding to a reasonable range for manipulating the intermediate representation.

For example, a case is also conceivable where the predetermined encoder E and the predetermined decoder D are of a second pattern. In this case, the objective function is, for example, used to train the predetermined encoder E and the predetermined decoder D and thereby train parameters, whereby the distribution of latent representations corresponding to the generated intermediate representation is trained. As a result, the distribution training unit 604 may obtain a distribution of latent representations corresponding to the intermediate expressions, the distribution of latent representations corresponding to a valid range for manipulating the intermediate expressions.

For example, a case may be considered in which a predetermined encoder E and a predetermined decoder D are of the third pattern. In this case, for example, the distribution training unit 604 uses an objective function to train a first parameter according to the predetermined encoder E based on the input data and also trains a second parameter according to the predetermined decoder D based on the first parameter. By training the second parameter, the distribution training unit 604 trains the distribution of each of the multiple latent representations represented by the second parameter. As a result, the distribution training unit 604 may obtain a distribution of latent representations corresponding to the intermediate expressions, which corresponds to a valid range for manipulating the intermediate expressions.

The sample selecting unit 605 selects samples of latent representations from the trained distribution according to the probability distribution. For example, a predetermined encoder E and a predetermined decoder D may be a first pattern. In this case, the sample selecting unit 605 obtains samples by, for example, sampling data according to the probability distribution indicated in the trained distribution. This allows the sample selecting unit 605 to obtain samples of latent representations that will serve as the basis for valid new intermediate representations.

Alternatively, for example, a predetermined encoder E and a predetermined decoder D may be a second pattern. In this case, for example, the sample selecting unit 605 selects as samples of latent representations, latent representations that in the distribution of latent representations represented by the trained parameters, are present within a range of ±1σ. This allows the sample selecting unit 605 to obtain samples of latent representations that will serve as the basis for valid new intermediate representations.

Alternatively, for example, a predetermined encoder E and a predetermined decoder D may be a third pattern. In this case, for example, the sample selecting unit 605 selects, a latent representation of one of the layers present within the range of ±1σ, for example, in the distribution of latent representations of one of the layers represented by one of the second parameters desired to be trained, as a sample of the latent representation of that one of the layers. This allows the sample selecting unit 605 to obtain a sample of a latent representation that will serve as the basis for a valid new intermediate representation.

The restoring unit 606 generates a new intermediate representation corresponding to the selected sample using a predetermined decoder D. For example, a case may be considered in which a predetermined encoder E and a predetermined decoder D are the first pattern. In this case, the restoring unit 606 generates a vector corresponding to the selected sample using, for example, a second multilayer perceptron in the predetermined decoder D. The restoring unit 606 restores multiple vectors representing the intermediate representation from a vector obtained by multiplying the generated vector by a second weight matrix using, for example, a second generator in the predetermined decoder D. This allows the restoring unit 606 to perform operations on the original intermediate representation within an operation range based on the distribution of the latent representation, thereby obtaining a valid, new intermediate representation.

Also, for example, a case where a predetermined encoder E and a predetermined decoder D are a second pattern is considered. In this case, the restoring unit 606 generates a new intermediate representation by, for example, repeatedly performing a deconvolution operation on the selected sample using a predetermined decoder D. This allows the restoring unit 606 to apply an operation to the original intermediate representation within an operation range based on the distribution of the latent representation, thereby obtaining a valid, new intermediate representation.

Also, for example, a case where a predetermined encoder E and a predetermined decoder D are a third pattern is considered. In this case, the restoring unit 606 repeatedly obtains a latent representation of the next higher layer by, for example, performing a deconvolution operation on the latent representation of one of the selected layers, starting from one layer. For example, the restoring unit 606 generates a new intermediate representation by performing a deconvolution operation on the latent representation of the top layer. This allows the restoring unit 606 to apply an operation to the original intermediate representation within an operation range based on the distribution of the latent representation, thereby obtaining a valid, new intermediate representation.

The output generating unit 607 uses the trained model M to generate output data that corresponds to the generated new intermediate representation. The output generating unit 607 generates output data, for example, by converting the generated new intermediate representation into output data using the decoder M_D of the trained model M. This allows the output generating unit 607 to obtain a variety of valid output data.

The output unit 608 outputs the processing results of at least one of the functional units. The output format may be, for example, display on a display, print out to a printer, transmission to an external device via the communications I/F 305, or storage to a storage device such as the memory 302 or disk 304 depicted in Fig. 3. This allows the output unit 608 to notify the user of the processing results of at least one of the functional units, thereby improving the convenience of the controller 201.

The output unit 608 outputs, for example, the output data generated by the output generating unit 607. For example, the output unit 608 transmits the generated output data to another computer. The other computer is, for example, the client device 202. For example, the output unit 608 outputs the generated output data so that it may be referenced by the user. This allows the output unit 608 to make valid output data available externally.

Here, while case has been described where the output generating unit 607 generates the output data using the trained model M, this is not a limitation. For example, the output generating unit 607 may not use the trained model M when generating the output data.

In this case, the storage unit 600 stores a first encoder E_1 that converts a vector into a latent representation and a first decoder D_1 corresponding to the first encoder E_1. The first encoder E_1 may be included in, for example, a processing request. The first decoder D_1 may be included in, for example, a processing request. The first decoder D_1 converts the latent representation into a vector. The first encoder E_1 is, for example, an encoder used in the VAE method. The first decoder D_1 is, for example, a decoder used in the VAE method. The first encoder E_1 is obtained by, for example, the obtaining unit 601. The first decoder D_1 is obtained by, for example, the obtaining unit 601.

The storage unit 600 stores, for example, a first model M_1 that converts an intermediate representation into a vector. The first model M_1 may be included in, for example, a processing request. The first model M_1 is, for example, an encoder in the AutoBot method. The first model M_1 is obtained by, for example, the obtaining unit 601. The storage unit 600 stores, for example, a second model M_2 that converts a vector into output data. The second model M_2 is, for example, a decoder in the AutoBot method. The second model M_2 is generated by, for example, the distribution training unit 604.

The obtaining unit 601 obtains, for example, the first encoder E_1 and the first decoder D_1. For example, the obtaining unit 601 obtains the first encoder E_1 and the first decoder D_1 by extracting the first encoder E_1 and the first decoder D_1 from the processing request. For example, the obtaining unit 601 may obtain the first encoder E_1 and the first decoder D_1 by receiving the first encoder E_1 and the first decoder D_1 from another computer. The other computer may be, for example, the client device 202. For example, the obtaining unit 601 may obtain the first encoder E_1 and the first decoder D_1 by receiving input of the first encoder E_1 and the first decoder D_1 based on a user's operation input via an input device (not depicted).

The obtaining unit 601 obtains, for example, the first model M_1. For example, the obtaining unit 601 obtains the first model M_1 by extracting the first model M_1 from the processing request. For example, the obtaining unit 601 may obtain the first model M_1 by receiving the first model M_1 from another computer. The other computer may be, for example, the client device 202. For example, the obtaining unit 601 may obtain the first model M_1 by receiving input of the first model M_1 based on a user's operation input via an input device (not depicted).

The intermediate representation generating unit 603 generates an intermediate representation corresponding to the input data using the trained model M. The intermediate representation generating unit 603 generates a first vector corresponding to the generated intermediate representation using the first model M_1. The intermediate representation generating unit 603 generates the first vector, for example, by converting the generated intermediate representation into the first vector using the first model M_1. This allows the intermediate representation generating unit 603 to obtain a first vector that extracts features of the input data.

The distribution training unit 604 uses the training data used when training the trained model M as the input data. The distribution training unit 604 sets an objective function including the likelihood of output data corresponding to input data and the KL divergence related to the distribution of latent representations. Using the set objective function, the distribution training unit 604 trains the distribution of latent representations corresponding to the generated first vector according to the first encoder E_1 and the first decoder D_1, and also trains the second model M_2. This allows the distribution training unit 604 to obtain the distribution of latent representations corresponding to the first vector. The distribution training unit 604 may prepare the second model M_2 so that output data may be generated.

The sample selecting unit 605 selects samples of latent representations from the trained distribution according to a probability distribution. This allows the sample selecting unit 605 to obtain samples of latent representations that will serve as the basis for a valid new vector.

The restoring unit 606 generates a new second vector, different from the first vector and corresponding to the selected samples, using the first decoder D_1. The restoring unit 606 generates a new second vector, for example, by converting the selected sample into a new second vector using the first decoder D_1. This allows the restoring unit 606 to operate on the original first vector within an operation range based on the distribution of the latent representation, thereby obtaining a valid new second vector.

The output generating unit 607 generates output data corresponding to the generated new second vector using the trained second model M_2. The output generating unit 607 generates output data, for example, by converting the generated new second vector into output data using the trained second model M_2. This allows the output generating unit 607 to obtain a variety of valid output data.

Here, while a case where the controller 201 includes the obtaining unit 601, the model training unit 602, the intermediate representation generating unit 603, the distribution training unit 604, the sample selecting unit 605, the restoring unit 606, the output generating unit 607, and the output unit 608 has been described, this is not a limitation. For example, the controller 201 may not include any of the functional units. For example, the controller 201 may not include the model training unit 602.

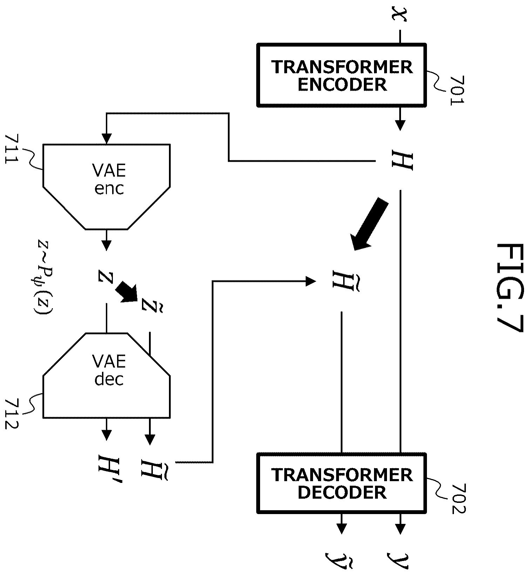

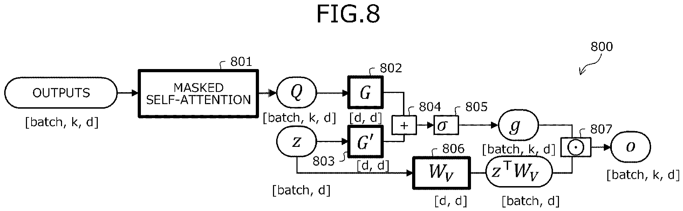

Next, a first operation example of the controller 201 will be described with reference to Figs. 7 and 8.

Figs. 7 and 8 are explanatory diagrams depicting the first operation example of the controller 201. In Fig. 7, the controller 201 has a trained model including an encoder 701, which is a Transformer encoder, and a decoder 702, which is a Transformer decoder. The controller 201 has a VAE model including a VAE encoder 711 and a VAE decoder 712. The VAE model has a function of reconstructing an intermediate representation. The intermediate representation is a set of T d-dimensional vectors. The controller 201 has training data used when training the trained model. In the following description, a character with ~ at the top may be written as "character ~."

The controller 201 sets training data to input data x. In the following description, the intermediate representation obtained by converting input data x using the encoder 701 may be written as "intermediate representation H." Furthermore, the output obtained by directly converting the intermediate representation H using the decoder 702 may be written as "output y." Furthermore, the latent representation obtained by converting the intermediate representation H using the VAE encoder 711 may be written as "latent representation z." Furthermore, the intermediate representation obtained by converting the latent representation z using the VAE decoder 712 may be written as "intermediate representation H'." Furthermore, the intermediate representation obtained by converting a sample z~ selected from the distribution Pψ(z) of the latent representation z using the VAE decoder 712 may be written as "intermediate representation H~." Furthermore, the output obtained by converting the intermediate representation H~ using the decoder 702 may be written as "output y~."

The controller 201 fixes the parameters of the trained model. The controller 201 sets an objective function. The objective function represents, for example, a weighted sum of the likelihood of the output y, the reconstruction error of the intermediate representation H, and the KL divergence with respect to the distribution Pψ(z) of the latent representation. Here, the VAE encoder 711 includes preprocessing in which T d-dimensional vectors serving as input intermediate representations are fully concatenated, and then a d-dimensional vector is generated by multiplying the T d-dimensional vectors by a (d×T)×d-dimensional weight matrix Wₑ. The VAE encoder 711 includes a multilayer perceptron that converts a d-dimensional vector into a d-dimensional vector.

The VAE decoder 712 is a model that includes a multilayer perceptron that converts a d-dimensional vector into a d-dimensional vector. The VAE decoder 712 includes a post-processing step that multiplies the d-dimensional vector by a d×(d×T)-dimensional weight matrix W_{d} to generate a d×T-dimensional vector and that restores T d-dimensional vectors serving as intermediate representations from the generated d×T-dimensional vector. The VAE decoder 712 may include a gating mechanism 800, described later in Fig. 8, that restores T d-dimensional vectors serving as intermediate representations from the d-dimensional vector output by the multilayer perceptron.

The controller 201 trains the distribution Pψ(z) of the latent representation z by training a VAE model using input data x to minimize the objective function. Training the VAE model corresponds to updating the VAE model. In the following description, the probability value of the latent representation z is assumed to be p(z). The controller 201 selects a sample z~ with a relatively high probability from the trained distribution Pψ(z). The controller 201 converts the selected sample z~ into an intermediate representation H~ using the VAE decoder 712. The controller 201 converts the converted intermediate representation H~ into an output y~ using the decoder 702.

This allows the controller 201 to obtain a variety of outputs y~ that differ from the output y obtained by directly converting the intermediate representation H using the decoder 702. The controller 201 may apply operations to the intermediate representation H within an operation range based on the distribution Pψ(z), thereby obtaining a valid, new intermediate representation H~ and a valid output y~. We now move on to the description of Fig. 8, which depicts an example of the gating mechanism 800.

In Fig. 8, the gating mechanism 800 determines an output o of [b,k,d] corresponding to the latent representation z of [b,d] according to past outputs Outputs of [b,k,d] and the latent representation z of [b,d]. b is the batch size. The gating mechanism 800 includes processing units 801 to 807. The gating mechanism 800 combines past outputs Outputs of [b,k,d] and the latent representation z of [b,d] via the processing units 801 to 807 to determine the output o of [b,k,d].

The processing unit 801 is masked self-attention. The processing unit 802 represents multiplication by a matrix G. The processing unit 803 represents multiplication by a matrix G'. The processing unit 804 represents addition. The processing unit 805 represents application of σ. Processing unit 806 represents multiplication by matrix W_{V}. Processing unit 807 represents element multiplication. For the gating mechanism 800, refer to Montero, Ivan, Nikolaos Pappas, and Noah A. Smith, "Sentence bottleneck autoencoders from transformer language models." arXiv preprint arXiv:2109.00055 (2021).

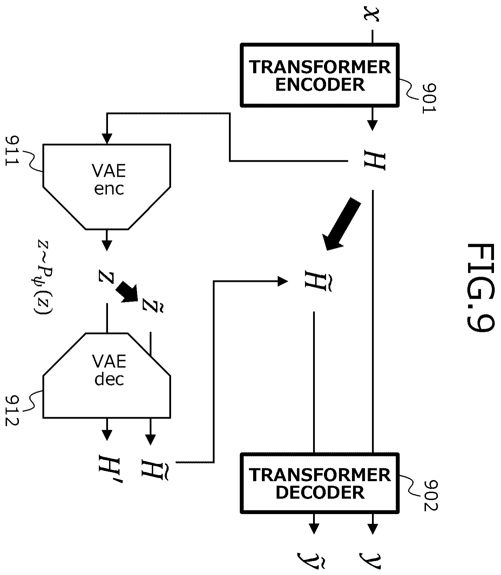

Next, a second operation example of the controller 201 will be described with reference to Figs. 9 to 12.

Figs. 9, 10, 11, and 12 are explanatory diagrams depicting a second operation example of the controller 201. In Fig. 9, the controller 201 has a trained model including an encoder 901, which is a Transformer-encoder, and a decoder 902, which is a Transformer-decoder. The controller 201 also has a VAE model including a VAE encoder 911 and a VAE decoder 912. The controller 201 also has training data used when training the trained model.

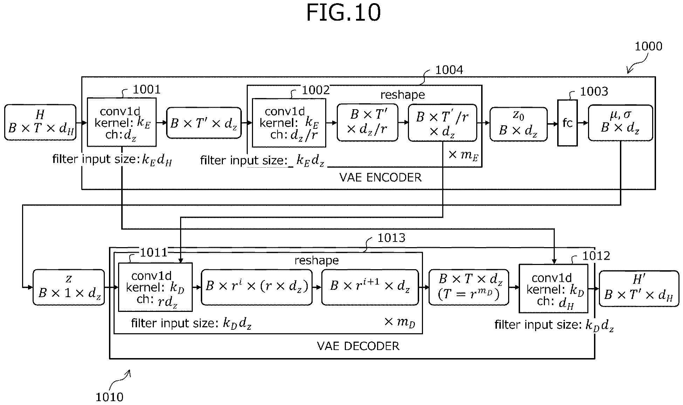

Here, because intermediate representations, which are the output of the Transformer, tend to be ultra-high-dimensional, it is considered preferable to recognize the intermediate representations as a series of vectors in the same space, capture information about the latent representations, and perform conversion into lower-dimensional representations, thereby making it easier to train the distribution of the latent representations. Therefore, the controller 201 applies a model including a convolution operation to the VAE encoder 911. For example, the controller 201 applies a model 1000, which will be described later with reference to Fig. 10, to the VAE encoder 911. Furthermore, the controller 201 applies a model including a deconvolution operation to the VAE decoder 912. For example, the controller 201 applies a model 1010, which will be described later with reference to Fig. 10, to the VAE decoder 912. Here, description is given with reference to Fig. 10.

Fig. 10 depicts an example of the model 1000 that serves as the VAE encoder 911, and an example of the model 1010 that serves as the VAE decoder 912. The model 1000 includes processing units 1001 to 1003. The model 1000 has the function of converting the intermediate representation H of B×T×d_{H} into a latent representation z₀ of B×d_{z} via the processing units 1001 to 1003, and also has the function of calculating parameters (µ,σ) of B×d_{z} that represent the distribution of the latent representation z₀.

The processing unit 1001 corresponds to a convolution operation that reduces the intermediate representation H of B×T×d_{H} to B×T'×d_{z}. The processing unit 1002 corresponds to a one-dimensional convolution operation in the sequence direction that reduces the number of channels by 1/r. The model 1000 includes a reshape operation that converts the output representation of B×T'×d_{z}/r obtained by the processing unit 1002 into B×T'/r×d_{z}. The model 1000 repeats m_{E} times a unit 1004 of processing consisting of the processing unit 1002 and the reshape operation. The processing unit 1003 estimates the distribution of the latent representation z₀.

The model 1010 includes processing units 1011 and 1012. The model 1010 has a function of converting a latent representation z of B×1×d_{z} selected from a distribution represented by the parameters (µ,σ) into an intermediate representation H' of B×T'×d_{H} via the processing units 1011 and 1012.

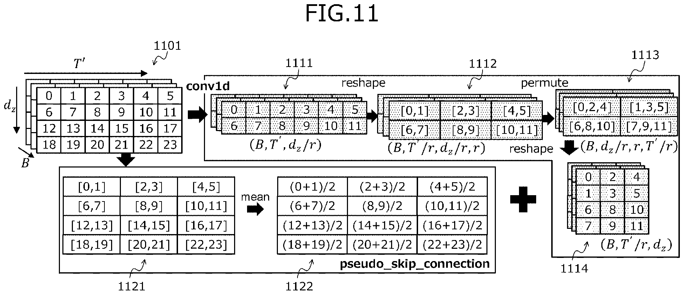

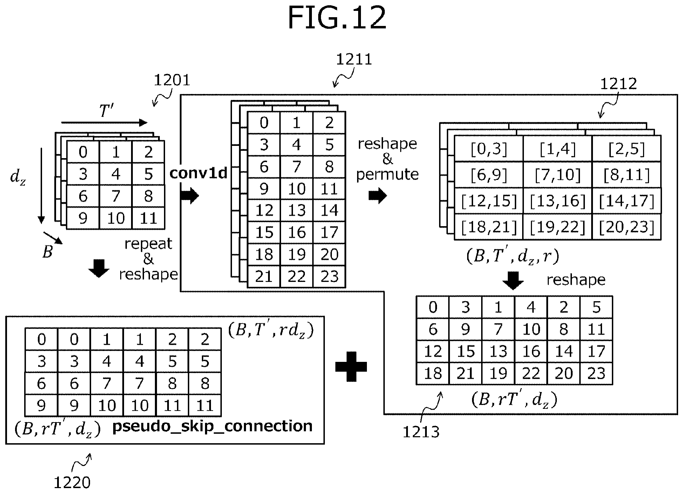

The processing unit 1011 corresponds to a one-dimensional deconvolution operation in the sequence direction that expands the number of channels by r times. The model 1010 includes, for example, a reshape operation that converts the output representation of B×r×(r×d_{z}) obtained by the processing unit 1011 into B×rⁱ⁺¹×d_{z}. The model 1010 repeats m_{D} times a unit 1013 of processing consisting of the processing unit 1011 and the reshape operation. The processing unit 1012 corresponds to a convolution operation that expands the output representation of B×T×d_{H} to B×T'×d_{H}. Next, a specific example of the units 1004 and 1013 of processing for a case where r=2 will be described with reference to Figs. 11 and 12.

Fig. 11 depicts a specific example of the unit 1004 of processing. In Fig. 11, conv1d is a process that converts an input of size (B×T×dᵢₙ) into an output of size (B×T×dₒᵤₜ) using a kernel of size k_{E} through a one-dimensional convolution operation. B is the batch size. T is the sequence length. dᵢₙ is the input channel size. dₒᵤₜ is the output channel size.

In Fig. 11, the input intermediate representation depicted in Table 1101 is (B,T',d_{z}). The values displayed in each rectangle are the component indexes. A common index is used for rectangles with the same background color. The intermediate representation depicted in Table 1101 is converted by conv1d into the output representation of (B,T',d_{z}/r) depicted in Table 1111.

The output representation depicted in Table 1111 is reshaped into the output representation of (B,T'/r,dz/r) depicted in Table 1112. The output representation depicted in Table 1112 is permuted into the output representation of (B,d_{z}/r,r,T'/r) depicted in Table 1113. The output representation depicted in Table 1113 is reshaped into the output representation of (B,T'/r,d_{z}) depicted in Table 1114.

For efficient training, it may be preferable to calculate the average of the output representation depicted in Table 1121, in which the input components are rearranged to have the same shape as the output, to generate and add the output representation depicted in Table 1122 to the output representation depicted in Table 1114. Here, description is given with reference to Fig. 12.

Fig. 12 depicts a specific example of the unit 1013 of processing. In Fig. 12, conv1d is a process that converts an input of size (B×T×dᵢₙ) into an output of size (B×T×dₒᵤₜ) using a kernel of size k_{D} through a one-dimensional deconvolution operation. B is the batch size. T is the sequence length. dᵢₙ is the input channel size. dₒᵤₜ is the output channel size.

In Fig. 12, the input output representation depicted in Table 1201 is (B,T',d_{z}). The values displayed in each rectangle are the component indexes. A common index is used for rectangles with the same background color. The intermediate representation depicted in Table 1201 is converted by conv1d into the output representation of (B,T',rd_{z}) depicted in Table 1211.

The output representation depicted in Table 1211 is reshaped and permuted to the output representation of (B,T'/r,d_{z},r) depicted in Table 1212. The output representation depicted in Table 1212 is reshaped to the output representation of (B,rT',d_{z}) depicted in Table 1213. For efficient training, it may be preferable to add the output representation depicted in Table 1220, which is obtained by repeating and reshaping the input, to the output representation depicted in Table 1213.

Here, description is given with reference to Fig. 9. The controller 201 has training data used when training the trained model. The controller 201 sets the training data to the input data x. The controller 201 fixes the parameters of the trained model. The controller 201 sets the objective function. The objective function represents, for example, a weighted sum of the likelihood of the output y, the reconstruction error of the intermediate representation H, and the KL divergence for the distribution Pψ(z) of the latent representation.

The controller 201 trains the distribution Pψ(z) of the latent representation z by training a VAE model using the input data x so as to minimize the objective function. Training the VAE model corresponds to updating the VAE model. In the following description, the probability value of the latent representation z is defined as p(z). The controller 201 selects a sample z~ with a relatively high probability from the trained distribution Pψ(z). The controller 201 converts the selected sample z~ into an intermediate representation H~ by repeatedly performing a deconvolution operation on the selected sample z~ using the VAE decoder 912. The controller 201 converts the converted intermediate representation H~ into an output y~ using the decoder 902.

This allows the controller 201 to obtain a variety of outputs y~ that differ from the output y obtained by directly converting the intermediate representation H using the decoder 902. The controller 201 may apply operations to the intermediate representation H within an operation range based on the distribution Pψ(z), thereby obtaining a new valid intermediate representation H~ and a valid output y~. The controller 201 may facilitate training the distribution of latent representations by utilizing a VAE model including a convolution operation.

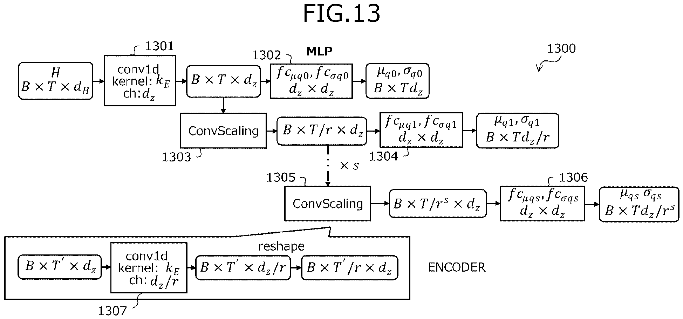

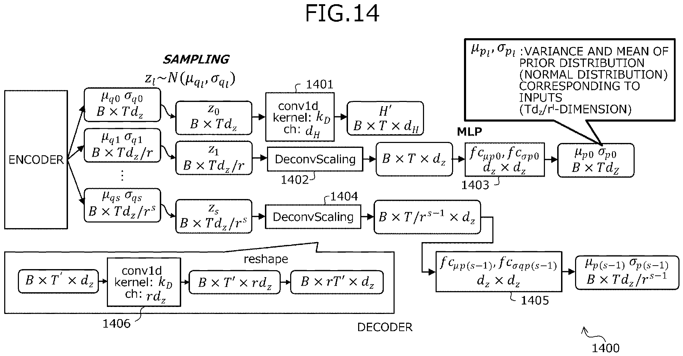

Next, a third operational example of the controller 201 will be described with reference to Figs. 13 and 14.

Figs. 13 and 14 are explanatory diagrams depicting a third operation example of the controller 201. As described above, the intermediate representations output by the Transformer tend to be ultra-high-dimensional, and it is considered preferable to recognize the intermediate representations as a series of vectors in the same space, capture information about the latent representations, and perform conversion into low-dimensional representations, thereby making facilitating training the distribution of the latent representations.

Thus, in the operation example 3, the controller 201 applies to the VAE encoder 911, a model that includes a convolution operation and that layers the latent representations. For details about layering the latent representations, refer to, for example, Child, Rewon, "Very deep vaes generalize autoregressive models and can outperform them on images." arXiv preprint arXiv:2011.10650

(2020). The latent representations are information extracted from the features of the intermediate representations at different levels of abstraction for each layer.

For example, the controller 201 applies a model 1300, which will be described later with reference to Fig. 13, to the VAE encoder 911. Furthermore, the controller 201 applies to the VAE decoder 912, a model that includes a deconvolution operation and that hierarchizes latent representations. For example, the controller 201 applies a model 1400, which will be described later with reference to Fig. 14, to the VAE decoder 912. Here, description is given with reference to Fig. 13.

Fig. 13 depicts an example of the model 1300 that serves as the VAE encoder 911. The model 1300 includes processing units 1301 to 1307 and the like. The model 1000 has a function of calculating posterior distribution parameters (µ_{ql},σ_{ql}) of B×Td_{z}/r^{l} representing the distribution of the latent representation zₗ at layer l from the intermediate representation H of B×T×d_{H} via the processing units 1301 to 1307 and the like. l=0, 1, ..., s.

The processing unit 1301 is conv1d. The processing unit 1302 estimates the distribution. The processing unit 1303 is ConvScaling. The processing unit 1304 estimates the distribution. The model 1000 includes multiple processing units similar to processing units 1303 and 1304, and repeats the one-dimensional convolution operation of ConvScaling. The processing unit 1305 is the lowest-level ConvScaling. The processing unit 1306 estimates the distribution. ConvScaling includes, for example, a processing unit 1307 and a reshape operation. For example, the model 1300 calculates the posterior distribution parameters (µ_{ql},σ_{ql}) for l=0, 1, ..., s. The posterior distribution parameters are, for example, the variance and mean of a normal distribution. Here, description is given with reference to Fig. 14.

Fig. 14 depicts an example of the model 1400 that serves as the VAE decoder 912. The model 1400 includes processing units 1401 to 1406, etc. The model 1400 has a function of converting, via the processing unit 1401, a latent representation z₀ of B×Td_{z} selected from the distribution represented by the posterior distribution parameters (µ_{ql},σ_{ql}) at l=0 into an intermediate representation H' of B×T×d_{H} The model 1400 has a function of converting a latent representation zₗ of B×Td_{z}/r^{l} selected from a distribution represented by the posterior distribution parameters (µ_{ql},σ_{ql}), where l=1, ..., s, via the processing units 1402 to 1406, etc.

The processing unit 1401 is conv1d. The processing unit 1402 is deconvscaling. The processing unit 1403 estimates the distribution. The model 1000 includes multiple processing units similar to processing units 1402 and 1403, and performs a one-dimensional convolution operation of deconvscaling on each layer. The processing unit 1404 is deconvscaling at the bottom layer. The processing unit 1405 estimates the distribution. Deconvscaling includes, for example, the processing unit 1406 and a reshape operation. For example, the model 1400 obtains prior distribution parameters (µₚₗ,σₚₗ) for l=0, 1, ..., s-1.

In the following description, it is assumed that the output is language. The controller 201 has training data used when training the trained model. The controller 201 sets the training data to the input data x. The controller 201 fixes the parameters of the trained model. The controller 201 sets the objective function λ_{NLL}NLL+λ_{MSE}MSE+ΣβₗKLDₗ. KLDₗ=KLD(qₗ∥pₗ)=-0.5(1+log(σ_{ql}²)-logσₚₗ²-(σ_{ql}²+(µ_{ql}-µₚₗ)²)/(σₚₗ²)). pₗ and qₗ are Td_{z}/r^{l}-dimensional normal distributions. NLL=Σᵢ^{T}(-log p(yᵢ)). T is the sequence length. p(yᵢ) is the model's prediction probability for the correct word yᵢ. MSE=Σi^{T}((∥Hᵢ^{'}-Hᵢ∥)²/(∥Hᵢ∥)²). Hᵢ and Hᵢ' are intermediate representations before and after restoration, and correspond to a position i in the sequence length T.

The controller 201 trains the prior distribution parameters (µₚₗ,σₚₗ) that represent the distribution of the latent representation zₗ at each layer l by training the VAE model using the input data x so as to minimize the objective function. Training the VAE model corresponds to updating the VAE model. The controller 201 selects a sample zₗ~ that belongs to a specific layer l and has a relatively high probability from the distribution represented by the prior distribution parameters (µₚₗ,σₚₗ) of any specific layer l. The specific layer l is, for example, the bottom layer. The controller 201 refers to the model 1400, performs a deconvolution operation on the sample zₗ~, and converts the sample zₗ~ into an output representation of the next higher layer, thereby converting the selected sample zₗ~ into an intermediate representation H~. The controller 201 converts the converted intermediate representation H~ into an output y~ using the decoder 902.

This allows the controller 201 to obtain a variety of outputs y~ that differ from the output y obtained by directly converting the intermediate representation H using the decoder 902. The controller 201 may apply operations to the intermediate representation H within an operation range based on the distribution Pψ(z), thereby obtaining a valid new intermediate representation H~ and a valid output y~. The controller 201 may easily train the distribution of latent representations by utilizing a VAE model including a convolution operation. Furthermore, the controller 201 may improve the expressive power of latent representations by layering the latent representations.

Here, while the specific layer l is the bottom layer, this is not a limitation. For example, the specific layer l may be a layer other than the bottom layer. Alternatively, the controller 201 may select multiple layers as the specific layer l. This allows the controller 201 to obtain valid outputs y~ via latent representations z~ of various levels of abstraction, thereby making it easier to obtain diverse outputs y~.

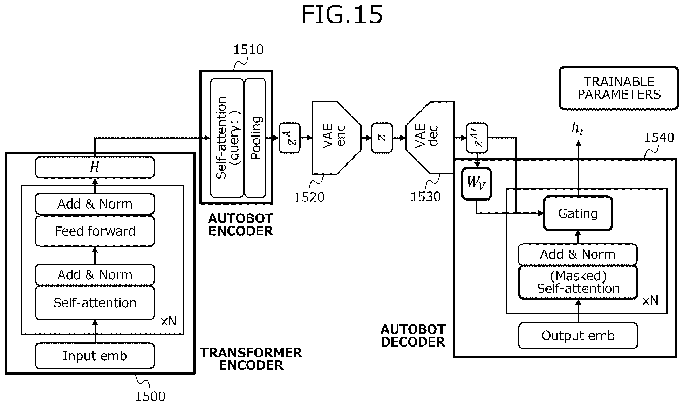

Next, a fourth operational example of the controller 201 will be described with reference to Fig. 15. This fourth operational example corresponds to a case in which the controller 201 does not use a trained model when generating output data.

Fig. 15 is an explanatory diagram depicting the fourth operational example of the controller 201. In Fig. 15, the controller 201 has a trained model encoder 1500. The controller 201 has an AutoBot encoder 1510. The controller 201 has a VAE model including a VAE encoder 1520 and a VAE decoder 1530. The controller 201 has an AutoBot decoder 1540. For details about AutoBot, refer to, for example, Montero, Ivan, Nikolaos Pappas, and Noah A. Smith, "Sentence bottleneck autoencoders from transformer language models". arXiv preprint arXiv: 2109.00055 (2021).

In the example depicted in Fig. 15, the controller 201 performs structure prediction directly from a latent representation z obtained from an intermediate representation H. The controller 201 specifies that the latent representation z^{A} generated by the encoder 1510 is projected to another latent representation z by the VAE encoder 1520, that the latent representation z^{A'} is reconstructed by the VAE decoder 1530, and that the latent representation z^{A'} is converted to an output ht by the decoder 1540.

The controller 201 has training data used when training the trained model. The controller 201 sets the training data to the input data x. The controller 201 fixes the parameters of the trained model. The controller 201 sets the objective function NLL+βKLD. KLD=-0.5(1+logo²-µ²-σ²). µ and σ are posterior distribution parameters. NLL=Σᵢ^{T}(-logp(yᵢ)). T is the sequence length. p(yᵢ) is the prediction probability of the model for the correct word yᵢ.

The controller 201 uses the input data x to train the VAE model so as to minimize the objective function, thereby training the posterior distribution parameters (µ,σ) and training the decoder 1540. Training the VAE model corresponds to updating the VAE model. Training the decoder 1540 corresponds to updating the decoder 1540. The controller 201 selects a sample z~ with a relatively high probability from the distribution represented by the posterior distribution parameters (µ,σ). The controller 201 converts the selected sample z~ into a latent representation z^{A}~ and converts the converted latent representation z^{A}~ into an output hₜ~ using the decoder 1540.

Thus, the controller 201 may obtain a variety of outputs ht~. The controller 201 may apply operations to the intermediate representation H within an operation range based on the distribution, and may obtain a valid output ht~.

As described, according to each operation example, the controller 201 may improve the quality of the output generation result obtained by operating on the intermediate representation. In the past, it was not clear whether the operated intermediate representation was within the range of the data distribution, and it was possible that an invalid output was obtained. In contrast, the controller 201 may identify operations on the intermediate representation to generate valid output by sampling through the latent space, thereby efficiently obtaining valid output.

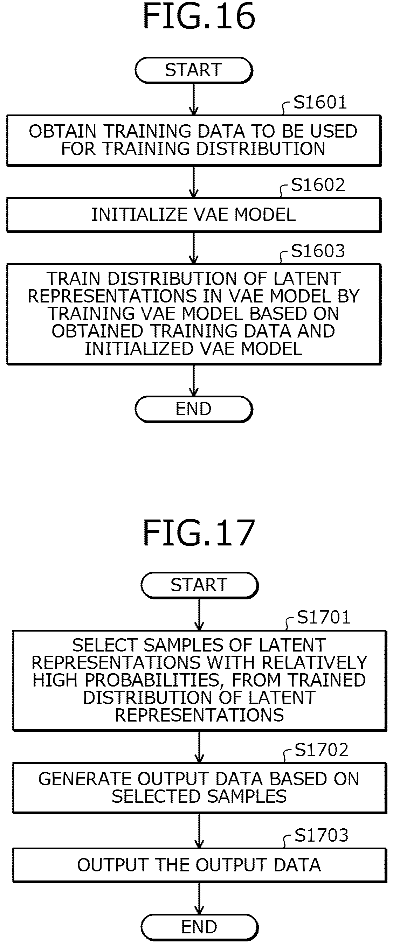

Next, an example of a procedure of a training process executed by the controller 201 will be described with reference to Fig. 16. The training process is implemented, for example, by the CPU 301 depicted in Fig. 3, storage devices such as the memory 302 and the disk 304, and the communications I/F 305.

Fig. 16 is a flowchart depicting an example of the procedure of the training process. In Fig. 16, the controller 201 obtains training data to be used for distribution training (step S1601). The training data does not necessarily have to be the same as the data used when training the trained model. The controller 201 initializes the VAE model (step S1602). As in either of the operation examples, the controller 201 trains the distribution of latent representations in the VAE model by training the VAE model based on the obtained training data and the initialized VAE model (step S1603). The controller 201 ends the training process.

Next, an example of a procedure of a generation process executed by the controller 201 will be described with reference to Fig. 17. The generation process is implemented, for example, by the CPU 301, storage devices such as the memory 302 and the disk 304, and the communications I/F 305 depicted in Fig. 3.

Fig. 17 is a flowchart depicting an example of the procedure of the generation process. In Fig. 17, the controller 201 selects samples of latent representations with relatively high probabilities from the distribution of trained latent representations (step S1701). As with any of the operation examples, the controller 201 generates output data based on the selected samples (step S1702). The controller 201 outputs the output data (step S1703). The controller 201 ends the generation process.

As described above, the controller 201 may generate intermediate representations corresponding to input data using a trained model. The controller 201 may train the distribution of latent representations corresponding to the generated intermediate representations according to a predetermined encoder that generates the latent representations and a predetermined decoder that corresponds to the predetermined encoder. The controller 201 may select samples of latent representations corresponding to a probability distribution from the trained distribution. The controller 201 may generate new intermediate representations corresponding to the selected samples using a predetermined decoder. The controller 201 may generate output data corresponding to the generated new intermediate representations using a trained model. This allows the controller 201 to obtain valid output data.

The controller 201 may employ the training data used when training the trained model as input data. The controller 201 may set an objective function that includes the likelihood of output data corresponding to input data, the reconstruction error of the intermediate representation corresponding to the input data, and the **KL** divergence related to the distribution of latent representations. The controller 201 may train the distribution of latent representations according to a predetermined encoder and a predetermined decoder using the objective function. This allows the controller 201 to accurately train the distribution of latent representations and easily obtain valid output data.

The controller 201 may utilize a predetermined encoder including a first generator and a first multilayer perceptron that generates a latent representation corresponding to the generated first vector. The first generator generates the first vector by fully concatenating multiple vectors representing the intermediate representation and then multiplying the vectors by a first weight matrix. The controller 201 may also have a predetermined decoder including a second multilayer perceptron that generates a second vector corresponding to the latent representation and a second generator. The second generator restores multiple vectors representing the intermediate representation from a third vector obtained by multiplying the generated second vector by the second weight matrix. This allows the controller 201 to utilize a corresponding combination of a predetermined encoder and a predetermined decoder to train the distribution of the latent representation.

The controller 201 may utilize a predetermined encoder that generates a latent representation by repeatedly performing a convolution operation on the intermediate representation and identifies parameters that represent the distribution of the latent representation. The controller 201 may utilize a predetermined decoder that generates an intermediate representation by repeatedly performing a deconvolution operation on the latent representation. The controller 201 may identify the distribution of latent representations by identifying the parameters. The controller 201 thus utilizes a corresponding combination of a predetermined encoder including a convolution operation and a predetermined decoder, thereby capturing information about latent representations and performing conversion into a lower-dimensional representation, making it easier to train the distribution of the latent representations.

The controller 201 may utilize a predetermined encoder including multiple repetitions of a convolution operation on an intermediate representation. The predetermined encoder makes it possible to identify a first parameter representing the distribution of each of multiple hierarchical latent representations corresponding to the results of each convolution operation. The controller 201 may utilize a predetermined decoder including performing a deconvolution operation on each of multiple latent representations. The predetermined decoder fixes a second parameter representing the distribution of the latent representation in the bottom layer and makes it possible to identify a second parameter representing the distribution of each of the latent representations other than the bottom layer. The controller 201 may train a first parameter based on input data according to a predetermined encoder, and train a second parameter based on the first parameter according to a predetermined decoder. The controller 201 may train the distribution of each of multiple latent representations represented by the second parameters. As a result, the controller 201 uses a corresponding combination of a predetermined encoder including a convolution operation and a predetermined decoder, thereby capturing information about the latent representations and performing conversion into a low-dimensional representation, making it easier to train the distribution of the latent representations.

The controller 201 may generate a first vector corresponding to the generated intermediate representation using a first model. The controller 201 may adopt the training data used in training the trained model as input data. The controller 201 may set an objective function including the likelihood of output data corresponding to input data and the **KL** divergence related to the distribution of the latent representations. The controller 201 may use the objective function to train the distribution of latent representations corresponding to the generated first vector according to the first encoder that generates the latent representations and the first decoder, and may train the second model that converts the vector into output data. The controller 201 may select samples of latent representations according to a probability distribution from the trained distribution. The controller 201 may generate a second vector corresponding to a selected sample using a first decoder. The controller 201 may generate output data corresponding to the generated second vector using the trained second model. This allows the controller 201 to generate output data without using a trained model.

The controller 201 may adopt a deep learning model that uses an amino acid sequence as input data and outputs output data representing a protein structure as the trained model. This allows the controller 201 to obtain output data representing a variety of valid protein structures.

The controller 201 may adopt a model that uses sequence information representing a sentence as input data and outputs sequence information representing another sentence as output data as the trained model. This allows the controller 201 to obtain output data representing a variety of valid sentences.

The control method described in the present embodiment may be implemented by executing a prepared program on a computer such as a personal computer and a workstation. The program is stored on a nontransitory, computer-readable recording medium such as a hard disk, a flexible disk, a compact disc read-only memory (CD-ROM), a magneto-optical (MO) disc, and a digital versatile disc (DVD), read out from the computer-readable medium, and executed by the computer. The program may be distributed through a network such as the Internet.

According to one aspect, it becomes possible to control the operation of intermediate representations in a direction that results in a valid output.

All examples and conditional language provided herein are intended for pedagogical purposes of aiding the reader in understanding the invention and the concepts contributed by the inventor to further the art, and are not to be construed as limitations to such specifically recited examples and conditions, nor does the organization of such examples in the specification relate to a showing of the superiority and inferiority of the invention. Although one or more embodiments of the present invention have been described in detail, it should be understood that the various changes, substitutions, and alterations could be made hereto without departing from the spirit and scope of the invention.

## Claims

1. A computer-readable recording medium storing therein a control program that causes a computer to execute a process, the process comprising:
generating an intermediate representation corresponding to input data, the intermediate representation being generated using a trained model;
training a distribution of latent representations corresponding to the generated intermediate representation, using a predetermined encoder generating the latent representation from the intermediate representation and a predetermined decoder corresponding to the predetermined encoder and generating an intermediate representation different from the intermediate representation;
selecting, from the trained distribution, a sample of the latent representation based on a probability distribution;
generating, using the predetermined decoder, a new intermediate representation corresponding to the selected sample; and
generating, using the trained model, output data corresponding to the generated new intermediate representation.

2. The control program according to claim 1, wherein
the training includes:
employing training data used in training the trained model as the input data; and
training the distribution of the latent representation according to the predetermined encoder and the predetermined decoder, using an objective function that includes a likelihood of output data corresponding to the input data, a reconstruction error of the intermediate representation corresponding to the input data, and a KL divergence related to the distribution of the latent representation.

3. The control program according to claim 2, wherein
the predetermined encoder includes:
a first generator that generates a first vector by fully concatenating a plurality of vectors representing the intermediate representation and then multiplying the fully concatenated vectors by a first weight matrix, and
a first multilayer perceptron that generates the latent representation corresponding to the generated first vector, and
the predetermined decoder includes:
a second multilayer perceptron that generates a second vector corresponding to the latent representation, and
a second generator that restores the plurality of vectors representing the intermediate representation from a third vector obtained by multiplying the generated second vector by a second weight matrix.

4. The control program according to claim 2, wherein
the predetermined encoder is a model that generates the latent representation by repeating a convolution operation on the intermediate representation and that identifies parameters representing a distribution of the latent representation,
the predetermined decoder is a model that generates the intermediate representation by repeating a deconvolution operation on the latent representation, and
the training includes identifying the distribution of the latent representation by identifying the parameters.

5. The control program according to claim 2, wherein
the predetermined encoder is a model that includes repeating a convolution operation on an intermediate representation a plurality of times and that identifies a first parameter representing the distribution of each of a plurality of hierarchical latent representations according to the results of each of the convolution operations,
the predetermined decoder is a model that includes performing a deconvolution operation on each of the plurality of latent representations and that fixes a second parameter representing the distribution of the latent representation in a bottom layer to identify a second parameter representing the distribution of each of the latent representations other than the bottom layer, and
the process of training includes:
training the first parameter according to the predetermined encoder based on the input data; and
training the second parameter according to the predetermined decoder based on the first parameter, thereby training the distribution of each of the latent representations represented by the second parameter.

6. The control program according to claim 1, the process further comprising:
generating a first vector corresponding to the generated intermediate representation using a first model;
adopting the training data used in training the trained model as the input data and utilizing an objective function including the likelihood of output data corresponding to the input data and the KL divergence related to the distribution of the latent representation, to train the distribution of latent representations corresponding to the generated first vector according to a first encoder generating the latent representation from the first vector and a first decoder corresponding to the first encoder and generating a vector different from the first vector and train a second model that converts the different vector into output data;
selecting a sample of the latent representation according to a probability distribution from the trained distribution; and
generating a second vector corresponding to the selected sample using the first decoder; and
generating output data corresponding to the generated second vector using the trained second model.

7. The control program according to any one of claims 1 to 6, wherein
the trained model is a deep learning model that, with an amino acid sequence as the input data, outputs the output data representing a protein structure.

8. The control program according to any one of claims 1 to 6, wherein
the trained model is a model that, with sequence information representing a sentence as the input data, outputs sequence information representing another sentence as the output data.

9. A control method executed by a computer, the method comprising:
generating an intermediate representation corresponding to input data, the intermediate representation being generated using a trained model;
training a distribution of latent representations corresponding to the generated intermediate representation, using a predetermined encoder generating the latent representation from the intermediate representation and a predetermined decoder corresponding to the predetermined encoder and generating an intermediate representation different from the intermediate representation;
selecting, from the trained distribution, a sample of the latent representation based on a probability distribution;
generating, using the predetermined decoder, a new intermediate representation corresponding to the selected sample; and
generating, using the trained model, output data corresponding to the generated new intermediate representation.

10. An information processing device, comprising:
a memory;
a processor coupled to the memory, the processor configured to:
generate an intermediate representation corresponding to input data, the intermediate representation being generated using a trained model;
train a distribution of latent representations corresponding to the generated intermediate representation, using a predetermined encoder generating the latent representation from the intermediate representation and a predetermined decoder corresponding to the predetermined encoder and generating an intermediate representation different from the intermediate representation;
select, from the trained distribution, a sample of the latent representation based on a probability distribution;
generate, using the predetermined decoder, a new intermediate representation corresponding to the selected sample; and
generate, using the trained model, output data corresponding to the generated new intermediate representation.
